# EUROPEAN PATENT SPECIFICATION

(11) **EP 2 961 726 B1**
(45) Date of publication and mention of the grant of the patent: **15.08.2018**
(21) Application number: 14706594.0
(22) Date of filing: 25.02.2014
(51) Int. Cl.: C07C 45/87, C07C 49/88, C07C 49/90

(54) **PROCESS FOR PREPARING KETENE IN THE PRESENCE OF A FLUIDIZED BED MATERIAL WITH A SURFACE AREA OF UP TO 600 M2/G**
VERFAHREN ZUR HERSTELLUNG VON KETEN IN GEGENWART EINES WIRBELBETTMATERIALS MIT EINER FLÄCHE BIS 600 M2/G
PROCÉDÉ DE PRÉPARATION DE CÉTÈNE EN PRÉSENCE D'UNE MATIÈRE POUR LIT FLUIDISÉ AVEC UNE SURFACE ATTEIGNANT 600 M2/G

(30) Priority: 27.02.2013 WO PCT/EP2013/053977; 28.02.2013 WO PCT/EP2013/054062; 25.03.2013 WO PCT/EP2013/056284
(43) Date of publication of application: 06.01.2016
(73) Proprietor: Haldor Topsøe A/S, 2800 Kgs. Lyngby (DK)
(72) Inventor: TAARNING, Esben, 2000 Frederiksberg (DK); HOLM, Martin Spangsberg, Oxford OX4 1BE (GB); OSMUNDSEN, Christian Mårup, 2820 Gentofte (DK)
(74) Representative: Haldor Topsøe A/S
(86) International application number: PCT/EP2014/053644
(87) International publication number: WO 2014/131764

(56) References cited:
- GB-A- 691 352
- JOHN C KAIG ET AL: "Ketene Formation from the Pyrolysis of Carbohydrates", SYMPOSIUM ON THERMAL USES AND PROPERTIES OF CARBOHYDRATES AND LIGNINS: PAPERS FROM THE SYMPOSIUM HELD AT THE 172ND NATIONAL MEETING OF THE AMERICAN CHEMICAL SOCIETY, ACAD. PRESS, USA; SAN FRANCISCO, CALIFORNIA, USA, 1 January 1976 (1976-01-01), pages 261-273, XP008166493, ISBN: 0-12-637750-2

## Description

Ketene (ethenone) is a highly volatile, reactive compound that has a tendency to react with other species, such as water, therefore making it difficult to detect and quantify.

GB 691,352 discloses a process of producing ketene on an industrial scale from a feedstock of acetone (or the corresponding ketone). The process may be conducted in a fluidized bed reactor using a bed of a ceramic material.

Symposium on thermal uses and properties of carbohydrates and lignins (1976) 261-273, discloses the formation of ketene from the pyrolysis of glucose via trapping experiments with deuterated water (deuterium oxide) and alkyl amines. A yield of 16% of ketene from the pyrolysis of glucose at 700 °C is disclosed; at lower reaction temperatures, such as 500 and 600 °C, the yield is 2.4 and 4.2 % respectively.

US 7,094,932 discloses the pyrolysis of an aqueous solution of glucose via a fluidized bed of sand. The condensable products are quantified; the aim of the experimental is to provide an improved process for preparing a glycolaldehyderich solution. Although US 7,094,932 cites 'Symposium on thermal uses and properties of carbohydrates and lignins (1976) 261-273' as disclosing the formation of ketene from glucose, no ketene product is observed in the products of the reactions disclosed in US 7,094,932, supporting the fact that pyrolysis reaction temperatures below 700 °C are not optimal for ketene (ethenone) formation.

Ind. Eng. Chem. Res. (1994) 2904 - 2912, discloses the use of chromatographic grade silica, i.e. high surface area silica, to increase the selectivity of ketene formation from acetates pre-adsorbed onto silica. At lower temperatures, 573 -673 K (300-400 °C), the selectivity for ketene formation from acetic acid (0.48-0.41) is higher than at higher temperatures, 773 - 973K (500-700 °C), (0.34-0.16). Reaction temperatures of 750 K were essential for steady state catalysis. When longer chain carboxylic acids are subjected to the reaction conditions, the longer chain ketene is formed; i.e. valeric acid forms propylketene. Smaller carbon chain compounds, e.g. propionic acid, provide the corresponding ketonisation product as the greatest yielding product.

It is therefore desirable to provide a high yielding, improved one-step catalytic process for the preparation of ketene (ethenone) from a feedstock comprising one or more sugars or glycolaldehyde. In particular, it is desirable to provide an improved process wherein ketene is obtainable in its free form and is suitable for subsequent transformations. In particular, the improved process provides a yield of ketene (ethenone) suitable for commercial viability of the process.

The process of the present invention is further defined as a process for preparing ketene from a feedstock in a fluidized bed reaction chamber, wherein the feedstock is pyrolysed in the presence of a fluidized bed material with a surface area between 200 and 600 m²/g.

According to the present invention the feedstock may be one or more sugars or glycolaldehyde, and the sugar is selected from one or more of the group consisting of glucose, fructose, galactose, xylose, sucrose and mannose. Preferably the feedstock is glucose. According to the present invention, the feedstock is introduced into the fluidized bed reaction chamber as an aqueous solution.

In a preferred embodiment of the present invention the fluidized bed material has an average particle size suitable for achieving a fluidized bed.

In a preferred embodiment of the present invention the fluidized bed material has a surface area of between 200 and 600 m²/g, preferably between 300 and 550 m²/g, more preferably between 400 and 600 m²/g.

In a preferred embodiment of the present invention the fluidized bed material has a pore volume of up to 0.80 ml/g, preferably between 0.40 and 0.75 ml/g, preferably between 0.50 and 0.70 ml/g, preferably 0.60 and 0.70 ml/g.

In a preferred embodiment of the present invention the fluidized bed material has a silanol concentration of up to 4.0 M, preferably between 2.0 M and 4.0 M, preferably between 3.0 and 4.5 M, preferably 3.5 M and 4.0 M.

In a preferred embodiment of the present invention the fluidized bed material is silicon oxide. In a more preferred embodiment the fluidized bed material is selected from the group consisting of high surface area silica.

In a preferred embodiment of the present invention the fluidized bed material is selected from the group consisting of SiC, silica gel and silica gel calcined at 500 °C.

In a preferred embodiment of the present invention the fluidized bed material is colloidal silica mixed with an oxide selected from the group consisting of Nb₂O₅, TiO₂, ZrO₂, CeO₂ and BaO.

In a preferred embodiment of the present invention the temperature of the reaction is less than 700 °C, preferably less than 600 °C, more preferably between 500 and 600 °C.
In a preferred embodiment of the present invention the reaction chamber contains a fluidized catalyst. More preferably the reaction chamber contains a fluidized catalyst and the residence time of the feedstock in the reaction chamber of the fluidized bed is between 50 to 150 ms.

In a preferred embodiment of the present invention the percentage yield of ketene is greater than 16 %.

In a preferred embodiment of the present invention the process is run as a continuous process.

In a preferred embodiment of the present invention the feedstock is introduced into the fluidized bed reaction chamber as an aqueous solution comprising up to 60% by weight of the feedstock, as disclosed in US 7,094,932. In a further preferred embodiment the feedstock is introduced into the fluidized bed reaction chamber as an aqueous solution comprising between 10% to 60% by weight of the feedstock. In a further preferred embodiment the feedstock is introduced into the fluidized bed reaction chamber as an aqueous solution comprising between 10% to 30% by weight of the feedstock. In a further preferred embodiment the feedstock is introduced into the fluidized bed reaction chamber as an aqueous solution comprising 10% by weight of the feedstock.

In a preferred embodiment of the present invention the feedstock is introduced into the fluidized bed reaction chamber as an atomized solution with a particle size of less than 10 µm.

In a preferred embodiment of the present invention the process is carried out under an inert atmosphere, e.g. an atmosphere of nitrogen.

### General Experimental:

The thermolytic conversion of carbohydrates was investigated in a fluidized bed setup. The setup consists of a stainless steel reactor (i.d.: 22 mm, length: 80 cm) fitted with a gas atomizing nozzle (Spraying Systems Co.), capable of delivering the liquid feed as a fine mist into the reactor (droplet size: <10 µm). The reaction temperature was monitored with a thermocouple extending from the top of the reactor into the bed. The top of the reactor was fitted with a disengager to prevent elutriation of the bed particles. Immediately after the outlet, the gas stream was directed through a condenser kept at 1 °C to collect the liquid product. The gas phase product may be passed into a subsequent reactor and transformed into further products as illustrated in 'Ketenes' Ullman's Encyclopedia of Industrial Chemistry (2002) 171-185.

In a typical experiment, the reactor was charged with 10 ml of the bed material, having a particle size from 90 to 150 µm. A nitrogen flow of 3.5 Nl/min was used to fluidize the bed, while the reactor temperature was raised to the desired reaction temperature of between 550-600 °C. When the reactor reached the desired temperature water was pumped to the nozzle, using a tube pump, at a flow rate 0.5 ml/min and injected into the bed. The liquid flow was maintained for at least 20 min to obtain a stable temperature in the bed.

The experiment was started by changing the liquid to a 10 wt % aqueous solution of the substrate, at which point the time was set as t = 0. The dead time from the feed flask to the nozzle was approx. 20 min. Collection of the liquid product was started at t = 30 min. The condensed liquid was collected over the entirety of the experiment to calculate mass balances. Each experiment was run for at least 6 hr.

Liquid products were quantified by HPLC analysis (Agilent, 1200 Series). The analytes were separated on a BioRad Aminex HPX-87H column operating at 65 °C. The eluent was a 0.005 M aqueous H₂SO₄, at a flow rate of 0.6 ml/min. The analytes were quantified using a RI detector against standard samples. Products were identified either by matching retention time with a known standard, or if possible by GC-MS analysis on an Agilent Technologies 6890 Plus series gas chromatograph with an Agilent Technologies 5973A series mass selective detector.

Analysis of the gas phase products was performed by directing part of the gas stream, after the condenser, into a mass spectrometer (IPI, GAM 200 Multi Component Gas Analyser).

### Measurement of fluidized bed material silanol concentration:

The dehydroxylation behavior of silica was measured on a Mettler TGA/DSC 1; the sample was dehydrated at 150 °C for 60 min in a flow of 20% O₂, 26% He and 54% Ar at 50 ml/min; this gas flow was maintained for the duration of the analysis. The temperature was lowered to 40 °C and then increased to 1500 °C at 5 °C /min, while monitoring the weight of the sample.

| | **BET surface are (m²/g)** | **Pore volume (ml/g)** | **Silanol concentration (M)** |
|---|---|---|---|
| Silica gel (SG60) | 517 | 0.68 | 3.60 |
| Silica gel (calcined at 1000 °C) | 4 | 0.01 | 0.20 |
| alpha-cristobalite | 4 | 0.01 | 0.00 |

The first two parameters (BET surface area and Pore Volume) are determined by N2 sorption. The silanol concentration is measured by a combination of TGA and Hg porosimetry.

### Example 1:

The experimental procedure was followed according to the general experimental, where the fluid bed material was a silica compound [α-cristobalite; Sigma Alrich named as Silicon dioxide (quartz, cristabolite), product number: 84878], the substrate was glucose (D-glucose monohydrate; Sigma Aldrich). The reactor temperature was 550 °C.

### Example 2:

The experimental procedure was followed according to the general experimental, where the fluidized bed material was a silica compound [silica gel; SG60 from Merck], the substrate was glucose (D-glucose monohydrate; Sigma Aldrich). The reactor temperature was 560 °C

### Example 3:

The experimental procedure was followed according to the general experimental, where the fluidized bed material was a silica compound [silica gel; SG60 from Merck) calcined at 500 °C], the substrate was glucose (D-glucose monohydrate; Sigma Aldrich).

### Example 4:

The experimental procedure was followed according to the general experimental, where the fluidized bed material was a silica compound [silica gel; SG60 from Merck], the substrate was glucose (D-glucose monohydrate; Sigma Aldrich). However, the reactor temperature was 515 °C.

### Example 5:

The experimental procedure was followed according to the general experimental, where the fluidized bed material was a silica compound [silica gel; SG60 from Merck], the substrate was glucose (D-glucose monohydrate; Sigma Aldrich). However, the reactor temperature was 590 °C.

### Example 6:

The experimental procedure was followed according to the general experimental, where the fluidized bed material was a silica compound [silica gel; SG60 from Merck], the substrate was glucose (D-glucose monohydrate; Sigma Aldrich). However, the liquid feed was a 30 wt % aqueous solution of the substrate, i.e. the concentration of the substrate in water was 30 wt % of glucose.

### Example 7:

The experimental procedure was followed according to the general experimental, where the fluidized bed material was prepared from a mixture of colloidal silica with a colloidal metal oxide selected from the group consisting of Nb₂O₅, TiO₂, ZrO₂, CeO₂ and BaO₂ (all colloidal metal oxides were obtained from Alfa Aesar). The colloidal silica and colloidal metal oxide mix [TiO₂, ZrO₂ or CeO₂] was prepared by mixing colloids of the desired oxides with colloidal silica (Ludox AS-30 from Sigma-Aldrich) in a ratio of 1:9 oxide to silica, and evaporating to dryness, followed by calcination at 1000 °C. An aqueous solution of BaNO₃ was used to prepare the BaO/colloidal silica mix. Nb₂O₅ was prepared by impregnation of NbCl₅ onto silica gel [SG60, Merck] by incipient wetness impregnation and calcined at 1000 °C.

**Table 1: Carbon yield and composition of the liquid condensate.**

| **Liquid Condensate Composition** | **Example** | | | | |
|---|---|---|---|---|---|
| | 1 | 2 | 4 | 5 | 6 |
| Glyoxal | 0.037 | 0.026 | 0.029 | 0.028 | 0.028 |
| Pyruvaldehyde | 0.065 | 0.088 | 0.110 | 0.080 | 0.090 |
| Glycolaldeyde | 0.582 | 0.217 | 0.292 | 0.213 | 0.385 |
| Formaldehyde | 0.067 | 0.098 | 0.090 | 0.092 | 0.072 |
| Acetic Acid | 0.007 | 0.024 | 0.018 | 0.021 | 0.011 |
| Acetol | 0.016 | 0.050 | 0.044 | 0.033 | 0.027 |

Table 2: Comparison of the MS signal of the gas phase components for Examples 1, 2, 4, 5 and 6 where the intensity of the MS signal of Examples 2, 4, 5 and 6 are normalized to the signal obtained in Example 1.

**Table 2 illustrates that an increase in surface area of the fluidized bed material increase the amount of acetone and ketene produced.**

| **Gas Phase MS signal** | **Example** | | | | |
|---|---|---|---|---|---|
| | 1 | 2 | 4 | 5 | 6 |
| Acetone (m/z = 58) | 1 | 6.25 | 2.9 | 5.3 | 7.6 |
| Ketene (m/z = 42) | 1 | 3.17 | 1.6 | 3.4 | 4.8 |

### Figures:

Figure 1: Carbon yield and product composition of liquid condensate of pyrolysis of 10 wt% aqueous solution of glucose according to Examples 1 and 2 wherein α-cristobalite or silica gel are used as fluidized bed material.
Figure 2: Carbon yield of condensed liquid products of Examples 2 [Silica gel as received] and 1 [silica gel calcined at 1000 °C is equivalent to α-cristobalite], as a function of time of the reaction.
Figure 3: Carbon yield and product composition of pyrolysis of 10 wt% aqueous solution of glucose according to Example 7 wherein colloids of acidic and basic oxides are mixed with colloidal silica and used as bed material.

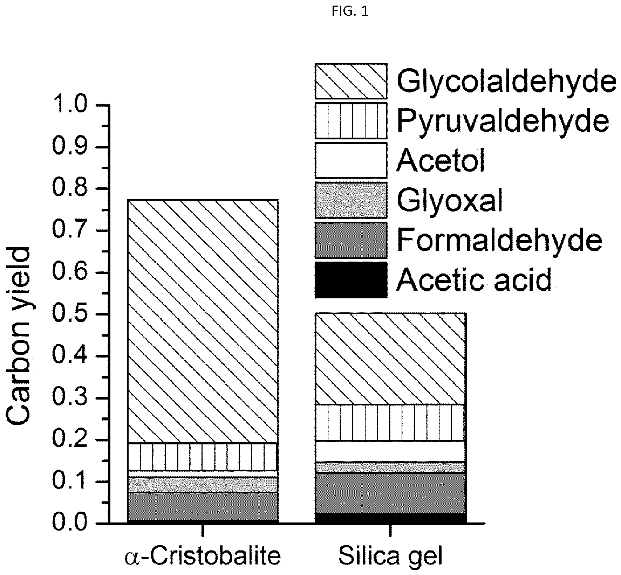

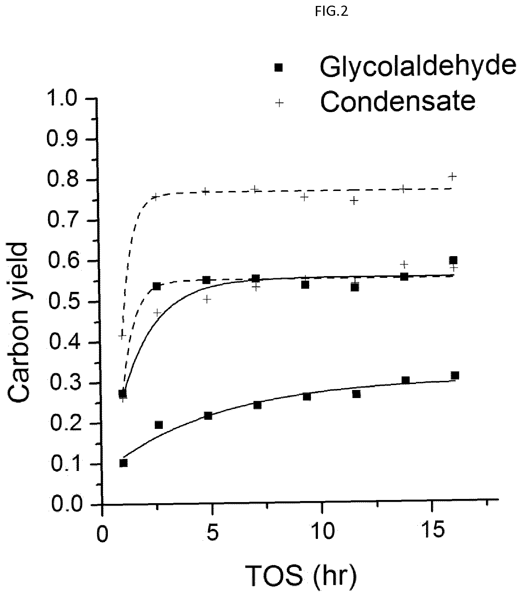

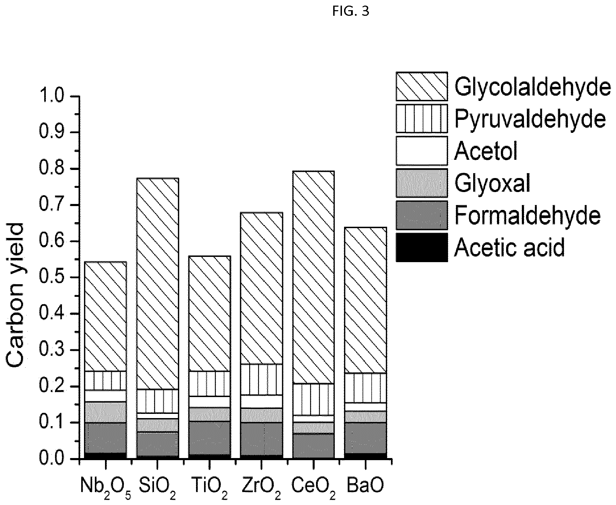

Figures 1 to 3 illustrate the increased yield of acetic acid produced as the surface area of the fluidized bed material increases. An increase in acetic acid yield correlates to an increase in ketene yield as ketene is hydrated to acetic acid.

## Claims

1. A process for preparing ketene from a feedstock in a fluidized bed reaction chamber, wherein the feedstock is pyrolysed in the presence of a fluidized bed material, wherein the surface area of the fluidized bed material is between 200 and 600 m² per gram and the feedstock is selected from one or more of the group consisting of glucose, fructose, galactose, xylose, sucrose and mannose and glycolaldehyde and the feedstock is introduced into the fluidized bed reaction chamber as an aqueous solution.

2. A process according to claim 1, wherein the fluidized bed material is silicon oxide.

3. A process according to claim 1, wherein the fluidized bed material is selected from the group consisting of SiC, silica gel, and silica gel calcined at 500 °C.

4. A process according to claim 1, wherein the fluidized bed material is colloidal silica mixed with an oxide selected from the group consisting of Nb₂O₅, SiO₂, TiO₂, ZrO₂, CeO₂ and BaO.

5. A process according to claims 1 to 4, wherein the temperature of the reaction is less than 700 °C.

6. A process according to claim 5, wherein the temperature of the reaction is between 500 and 600 °C.

7. A process according to claims 1 to 6, wherein the residence time of the material in the reaction chamber of the fluidized bed is up to 150 ms.

8. A process according to claims 1 to 7, wherein the percentage yield of ketene is greater than 16 %.

9. A process according to claims 1 to 8, wherein the feedstock is introduced into the fluidized bed reaction chamber as an aqueous solution comprising up to 60% by weight of feedstock.

10. A process according to claims 1 to 9, wherein the feedstock is introduced into the fluidized bed reaction chamber as an atomized solution with a particle size of less than 10 µm.

11. A process according to claims 1 to 10, wherein the process is carried out under an inert atmosphere of nitrogen.

12. A process according to claims 1 to 11, wherein the ketene is ethenone.

## Patentansprüche

1. Verfahren zur Herstellung von Keten aus einem Einsatzmaterial in einer Wirbelschichtreaktionskammer, wobei das Einsatzmaterial in Gegenwart eines Wirbelschichtmaterials pyrolysiert wird, wobei die Oberfläche des Wirbelschichtmaterials zwischen 200 und 600 m² pro Gramm beträgt und das Einsatzmaterial ausgewählt ist unter einem oder mehreren aus der Gruppe bestehend aus Glucose, Fructose, Galactose, Xylose, Saccharose und Mannose und Glycolaldehyd und das Einsatzmaterial in die Wirbelschichtreaktionskammer in Form einer wässrigen Lösung eingebracht wird.

2. Verfahren gemäß Anspruch 1, wobei das Wirbelschichtmaterial Siliziumoxid ist.

3. Verfahren gemäß Anspruch 1, wobei das Wirbelschichtmaterial ausgewählt ist aus der Gruppe bestehend aus SiC, Silicagel und bei 500 °C kalziniertem Kieselgel.

4. Verfahren gemäß Anspruch 1, wobei das Wirbelschichtmaterial kolloidales Siliciumdioxid ist, das mit einem Oxid, ausgewählt aus der Gruppe bestehend aus Nb₂O₅, SiO₂, TiO₂, ZrO₂, CeO₂ und BaO, gemischt ist.

5. Verfahren gemäß den Ansprüchen 1 bis 4, wobei die Reaktionstemperatur weniger als 700 °C beträgt.

6. Verfahren gemäß Anspruch 5, wobei die Reaktionstemperatur zwischen 500 und 600 °C liegt.

7. Verfahren gemäß den Ansprüchen 1 bis 6, wobei die Verweilzeit des Materials in der Wirbelschichtreaktionskammer bis zu 150 ms beträgt.

8. Verfahren gemäß den Ansprüchen 1 bis 7, wobei die prozentuale Ketenausbeute größer als 16 % ist.

9. Verfahren gemäß den Ansprüchen 1 bis 8, wobei das Einsatzmaterial in Form einer wässrigen Lösung, die bis zu 60 Gew.-% Einsatzmaterial enthält, in die Wirbelschichtreaktionskammer eingebracht wird.

10. Verfahren gemäß den Ansprüchen 1 bis 9, wobei das Einsatzmaterial in die Wirbelschichtreaktionskammer in Form einer zerstäubten Lösung mit einer Teilchengröße von weniger als 10 µm eingeführt wird.

11. Verfahren gemäß den Ansprüchen 1 bis 10, wobei das Verfahren unter einer Inertgasatmosphäre aus Stickstoff durchgeführt wird.

12. Verfahren gemäß den Ansprüchen 1 bis 11, wobei das Keten Ethenon ist.

## Revendications

1. Procédé de préparation de cétène à partir d'une charge d'alimentation dans une chambre de réaction à lit fluidisé, la charge d'alimentation étant pyrolysée en présence d'une matière pour lit fluidisé, l'aire surfacique de la matière pour lit fluidisé étant située entre 200 et 600 m² par gramme et la charge d'alimentation étant choisie parmi un ou plusieurs du groupe constitué par le glucose, le fructose, le galactose, le xylose, le saccharose et le mannose et le glycolaldéhyde et la charge d'alimentation étant introduite dans la chambre de réaction à lit fluidisé sous forme de solution aqueuse.

2. Procédé selon la revendication 1, la matière à lit fluidisé étant de l'oxyde de silicium.

3. Procédé selon la revendication 1, la matière pour lit fluidisé étant choisie dans le groupe constitué par SiC, le gel silicique et le gel silicique calciné à 500°C.

4. Procédé selon la revendication 1, la matière pour lit fluidisé étant de la silice colloïdale mélangée avec un oxyde choisi dans le groupe constitué par Nb₂O₅, SiO₂, TiO₂, ZrO₂, CeO₂ et BaO.

5. Procédé selon les revendications 1 à 4, la température de réaction étant inférieure à 700°C.

6. Procédé selon la revendication 5, la température de réaction étant située entre 500 et 600°C.

7. Procédé selon les revendications 1 à 6, le temps de séjour de la matière dans la chambre de réaction à lit fluidisé étant de jusqu'à 150 ms.

8. Procédé selon les revendications 1 à 7, le pourcentage de rendement en cétène étant supérieur à 16%.

9. Procédé selon les revendications 1 à 8, la charge d'alimentation étant introduite dans la chambre de réaction à lit fluidisé sous forme d'une solution aqueuse comprenant jusqu'à 60% en poids de charge d'alimentation.

10. Procédé selon les revendications 1 à 9, la charge d'alimentation étant introduite dans la chambre de réaction à lit fluidisé sous forme d'une solution atomisée présentant une grosseur de particule inférieure à 10 µm.

11. Procédé selon les revendications 1 à 10, le procédé étant effectué sous une atmosphère inerte d'azote.

12. Procédé selon les revendications 1 à 11, le cétène étant l'éthénone.
